# EUROPEAN PATENT APPLICATION

(11) **EP 4 570 232 A1**
(43) Date of publication of application: **18.06.2025**
(21) Application number: 23216828.6
(22) Date of filing: 14.12.2023
(51) Int. Cl.: A61K 8/02, A61K 8/25, A61K 8/39, A61K 8/60, A61Q 11/00

(54) **POWDER WITH SURFACTANTS FOR USE IN TREATING TOOTH SURFACES**

(71) Applicant: Ferton Holding S.A., 2800 Delémont (CH)
(72) Inventor: Donnet, Marcel, 01530 St. Jean de Gonville (FR); Gatti, Simone, 1022 Chavannes-prés-Renens (CH); Kratky, Julia, 64673 Zwingenberg (DE)
(74) Representative: Müller Schupfner & Partner Patent- und Rechtsanwaltspartnerschaft mbB (Muc)

(57) **Abstract**

A powder for use in treating tooth surfaces with a powder jet device is provided, wherein the powder comprises 0.1 - 2.0 wt.-% silica and 0.2 - 1.5 wt.-% of one or more surfactant(s).

## Description

The invention relates to a powder for use in treating tooth surfaces with a powder jet device. The invention further relates to the use of the powder in a powder jet device.

Professional dental prophylaxis is a maintenance treatment comprising powder jet cleaning or air polishing and aims to remove dental plaque and calculus. Such powder jet cleaning is particularly effective as it allows to reach and clean all tooth surfaces and the interspaces between them as well as implants, brackets and appliances. The professional dental prophylaxis is an important treatment that helps to maintain oral health since it removes the biofilm and calculus that a patient cannot remove during his daily home dental care.

In the powder jet cleaning process, a powder jet device is used, wherein a powder is sprayed with a gaseous carrier medium, usually air, onto tooth surfaces, which allows an efficient cleaning of the teeth. Additionally or as an alternative to a gaseous carrier medium, a liquid carrier medium, for example water may be used. The liquid carrier medium may surround the powder jet. Powder jet cleaning is performed with a powder jet device and it is particularly effective because it does not require repetitive movements nor different stages. Further, it is faster than other cleaning methods and it needs relatively low training to be learned correctly.

Prophylaxis powders are used worldwide and need to work reliably. Small amounts of silica, in particular hydrophobic silica are usually added to the powder to get a good flowability of the powder and to protect the powder against humidity. A disadvantage is that hydrophobic silica creates a so-like foam inside the patient mouth which is disturbing for the practitioner during the treatment. Remaining silica on the teeth may impair the bonding efficiency of subsequent dental treatments. Furthermore, when sucking out all powders and residues within the dental chair suction unit, these hydrophobic particles can deposit in the suction duct and overtime clog the suction unit.

It is therefore desired to have the powder protected against humidity and moisture and at the same time to give to the powder a hydrophilic behaviour so that the powder can be easily removed from the mouth of the patient by suction and pass through the suction ducts without leaving residues.

Therefore, it is an object of the present invention to provide a powder for use in treating tooth surfaces with a powder jet device that is protected against moisture and can easily be removed from the mouth of a patient by suction without leaving residues.

This object is achieved according to the invention by a powder for use in treating tooth surfaces with a powder jet device according to claim 1. The object is also achieved by the use of the powder according to claim 12. Preferred embodiments of the invention are subject to the dependent claims as well as the following description and Figures. According to the invention, treating tooth surfaces essentially means cleaning tooth surfaces.

According to one embodiment of the invention, a powder is provided for use in treating tooth surfaces with a powder jet device, wherein the powder comprises 0.1 - 2.0 wt.-% silica, based on the total weight of the powder, and wherein the powder further comprises 0.2 - 1.5 wt.-% of one or more surfactant(s), based on the total weight of the powder.

It has surprisingly been found that the powder according to the invention is protected against moisture by the silica and that the surfactant imparts the powder a hydrophilic character so that the powder can easily be removed by suction from the mouth of the patient together with the water that is used during the powder jet cleaning of the tooth and no residues clog the suction ducts.

A powder according to the invention means a substance in the form of small particles. The average particle size (d₅₀) of the powder according to the invention is preferably 0,5 to 500 µm, more preferably 1 - 300 µm, even more preferably 2 to 200 µm, most preferably 3 to 100 µm. The particle sizes may be adapted to the field of application. For example, a smaller average particle size of the particles is preferred for treating subgingival tooth surfaces, in particular about 0,5 to 50 µm, more preferably 1 to 30 µm. For treating supragingival tooth surfaces, a larger average particle size can be used, preferably 1 to 500 µm, more preferably 2 to 200 µm.

In the context of this invention, the d₅₀ value is the particle size at which 50 % of the particles are smaller than the d₅₀ value in terms of volume and 50 % of the particles are larger than the d₅₀ value in terms of volume. This applies also to the d₉₉ values where 99 % of the particles are smaller than the d₉₉ value in terms of volume. The d-values according to the invention are determined by laser diffraction method using a dry dispersion unit (Malvern Mastersizer 2000, equipped with a Scirocco dry dispersion unit, operated at 1.5 bar).

The d₉₉ of the powder according to the invention is preferably < 180 µm, more preferably < 150 µm, even more preferably < 120 µm.

A surfactant according to the invention is a chemical compound that decreases the surface tension or interfacial tension between two liquids, a liquid and a gas, and/or a liquid and a solid. Surfactants may function for example as emulsifiers, wetting agents, detergents, foaming agents, or dispersants. Preferably, a surfactant according to the invention is understood to be not a bactericide (disinfectant).

Surfactants can further be characterized by the hydrophilic-lipophilic balance (HLB) which is an indicator of its degree of hydrophilicity or lipophilicity, determined by calculating percentages of molecular weights for the hydrophilic or lipophilic portions in the surfactant molecule. The HLB according to the invention is calculated by the following formula (Griffin's method): HLB = 20 x Mₕ/M, wherein Mₕ is the molecular mass of the hydrophilic portion of the surfactant (surfactant molecule), and M is the molecular mass of the entire molecule (total molecular mass of the surfactant (surfactant molecule)). The result is a scale from 0 to 20. A HLB value of 0 means that the surfactant is completely lipophilic/hydrophobic and a value of 20 means that the molecule is completely hydrophilic/lipophobic.

The HLB value can be used to specify surfactants according to the following:
- < 10: Lipid-soluble (water-insoluble)
- > 10: Water-soluble (lipid-insoluble)
- 1 to 3: anti-foaming agent
- 3 to 6: W/O (water in oil) emulsifier
- 7 to 9: wetting and spreading agent
- 13 to 16: detergent
- 8 to 16: O/W (oil in water) emulsifier
- 16 to 18: solubilizer or hydrotrope

The powder according to invention preferably comprises 0.25 - 1.2 wt.-% of the one or more surfactant(s), even more preferably 0.3 - 1.1 wt.-% and most preferably 0.4 - 1.0 wt.-% of the one or more surfactant(s), based on the total weight of the powder.

The powder according to invention comprises preferably 0.1 - 2.0 wt.-%, more preferably 0.2 - 1.8 wt.-%, most preferably 0.3 - 1.7 wt.-% silica, based on the total weight of the powder.

Silica according to the invention is silicon dioxide which can be modified, in particular on the surface. Usual silica is hydrophilic so that usual silica can be named hydrophilic silica. Hydrophobic silica is silica with hydrophobic groups chemically bond to the surface of the silica. Examples of hydrophobic groups are alkyl or alkyl-polydimethylsiloxane groups. Silica according to the invention in particular comprises hydrophobic silica and hydrophilic silica.

In a preferred embodiment of the invention, the silica is a mixture of hydrophobic silica and hydrophilic silica, more preferably a mixture of hydrophobic silica and hydrophilic silica in a mass ratio of 10:1 to 1:3 (hydrophobic silica:hydrophilic silica), even more preferably a mixture of hydrophobic silica and hydrophilic silica in a mass ratio of 3:1 to 1:1 (hydrophobic silica:hydrophilic silica), most preferably a mixture of hydrophobic silica and hydrophilic silica in a mass ratio of about 3:2.

In order to improve the powder properties, the silica, preferably amorphous silica, comprises hydrophobic silica. This repels the humidity from the powder and ensures a good powder behaviour within the expiry term of the powder. Hydrophobic components in the powder, in particular hydrophobic silica has the problem that it makes the entire powder not soluble in water so that a foam in the mouth of the patient is formed which also makes the removal through suction difficult and the residues can clog the suction ducts by leaving residues. This problem is often encountered by reducing the amount of the hydrophobic silica and to add hydrophilic silica in order to ensure good flowability of the powder. Reducing the amount of hydrophobic silica, however, makes the powder being less protected against humidity. The result is that the powder has other disadvantages such as it absorbs humiditiy which deteriorates the rheology so that no powder is coming out from the powder chamber of the powder jet device when it stays there for a few days. This occurs often in the summer or when the humidity is high and it makes handling of the powder difficult.

This problem is solved by the powder according to the invention wherein the surfactant enables the solubility of the entire powder, including possible hydrophobic silica so that the powder can easily be removed from the mouth of the patient by suction. Further, no unpleasant foam in the mouth of the patient is formed which disturbs the practitioner during the cleaning process of the tooth.

The powder for use in treating tooth surfaces with a powder jet device according to the invention comprises 0.1 - 2.0 wt.-% silica, based on the total weight of the powder, and 0.2 - 1.5 wt.-% of one or more surfactant(s), based on the total weight of the powder.

The surfactant preferably has a hydrophilic-lipophilic balance (HLB) of 3 - 20, more preferably 5 - 19, even more preferably 7 - 18, even more preferably 8 - 17, even more preferably 9 - 16, most preferably 10 - 15. The HLB is calculated according to the formula HLB = 20 x Mₕ/M, wherein Mₕ is the molecular mass of the hydrophilic portion of the molecule, and M is the total molecular mass of the molecule.

In a preferred embodiment of the invention, the powder comprises one or more components selected from the group consisting of sodium hydrogen carbonate, amino acids, alditols, sugars and cyclodextrins.

In another preferred embodiment of the invention, the powder comprises 70 - 99 wt.-%, more preferably 80 - 98 wt.-%, even more preferably 85 - 96,5 wt.-%, based on the total weight of the powder, of one or more components selected from the group consisting of sodium hydrogen carbonate, amino acids, alditols, sugars and cyclodextrins.

The alditol according to the invention is preferably selected from the group consisting of erythritol, sorbitol, xylit (xylitol), mannit (mannitol), lactitol, threit (threitol), arabitol, and isomalt. More preferred are mannitol and erythritol. Most preferred is erythritol.

The amino acid is preferably glycine. The sugar is preferably trehalose, tagatose, rhamnose or arabinose.

In a preferred embodiment of the invention, the powder comprises of one or more components selected from the group consisting of sodium hydrogen carbonate, glycine, mannitol, erythritol, trehalose, tagatose, rhamnose and arabinose.

In another preferred embodiment of the invention, the powder comprises 70 - 99 wt.-%, more preferably 80 - 98 wt.-%, even more preferably 85 - 96,5 wt.-%, based on the total weight of the powder, of one or more components selected from the group consisting of sodium hydrogen carbonate, glycine, mannitol, erythritol, trehalose, tagatose, rhamnose and arabinose.

The surfactants according to the invention can be non-ionic, cationic, anionic or amphoteric. Preferred surfactants are:

### Non-ionic surfactants

PEG based :
- Tween 20, Tween 65, Tween 80, wherein Tween 65 is preferred
- Span 20, Span 40, Span 80, Span 83
- Pluronic: F-108, P-123, F-127, L-61, L-121, wherein P-123, , L-61, and L-121 are preferred
- BRIJ: S2, L4, C10, O20, L23, 58, S100, wherein S2, L4, C10, and O20 are preferred, L4 being most preferred

A preferred polyethylene glycol based surfactant is polyethylene glycol dodecylether (polyethylene glycol laurylether) according to the formula HO(CH₂CH₂O)ₚ-n-C₁₂H₂₅, wherein p = 2 -10, preferably p = 3 - 6, more preferably p = 4. Polyethylene glycol dodecylether with p = 4 is also named L4.
Glucoside based:
   - Capryl Glucoside, Decyl Glucoside, Coco Glucoside, Lauryl Glucoside, wherein Capryl Glucoside and Decyl Glucoside are preferred, Decyl Glucoside is more preferred, in particular n-Decyl Glucoside.
Alcohol based:
   Pentanol, Hexanol, Stearyl alcohol, Cetyl alcohol
Polypropylene glycol:
   PPG 1000, PPG 400, wherein PPG 1000 is preferred
Other:
   Laonine
Ionic surfactants:
   Acids:
   - Palmitic acid, arachidic acid, decanoic acid, wherein decanoic acid is preferred
Other:
   - Sodium dodecyl sulphate, Sodium coco sulphate, Tocopherol succinate

The surfactant can be added preferably as fine particles (for example Pluronic F-108) or absorbed in a carrier, preferably porous silica (for example Syoid XDP 3050) or maltodextrin. With the absorption in porous media, also liquid surfactants can be used as an additive. In a preferred embodiment of the invention, the powder comprises the surfactant absorbed in silica, preferably absorbed in mesoporous silica, more preferably hydrophilic silica, most preferably mesoporous hydrophilic silica. "Absorbed in" according to the invention means "absorbed in" and/or "absorbed on" and can also be described as "embedded in".

In a preferred embodiment of the invention, the powder additionally comprises a flow aid, a bleaching agent, a bactericide, an analgesic and/or a flavouring agent. The total amount of these additional substances is preferably 0.3 to 5 wt.-%, based on the total weight of the powder, more preferably 0.5 to 2 wt.-%.

Within the meaning of the present invention it should be understood that the amounts of the components of the powder, given in wt.-% (weight-%), sum up to 100 %. By way of example, a powder comprising 2 wt.-% amorphous silicon dioxide contains other components in a total amount of 98 wt.-%.

Preferred bleaching agents are peroxides such as magnesium, calcium or zinc peroxides, persulfates such as sodium, potassium or ammonium persulfates or perborates. Preferred bactericides are chlorhexidine, chlorhexidine digluconate, triclosan, cetylpyridinium chloride, hexetidine, tin(II) salts and zinc salts. Preferred analgesics are lidocaine and articaine.

In a preferred embodiment of the invention, the powder comprises less than 0.3 wt.-%, more preferably less than 0.2 wt.-%, even more preferably less than 0.1 wt.-% and most preferably less than 0.05 wt.-% chlorhexidine, chlorhexidine digluconate and/or cetylpyridinium chloride, based on the total weight of the powder. Most preferably, the powder according to the invention contains no chlorhexidine, chlorhexidine digluconate and/or cetylpyridinium chloride.

In another preferred embodiment of the invention, the powder comprises less than 0.3 wt.-%, more preferably less than 0.2 wt.-%, even more preferably less than 0.1 wt.-% and most preferably less than 0.05 wt.-% bactericide (disinfectant), based on the total weight of the powder. Most preferably, the powder according to the invention contains no bactericide.

The invention also relates to the use of the powder according to the invention in treating tooth surfaces or applying the powder to tooth surfaces. The invention further relates to the use of the powder according to the invention in a powder jet device. This use comprises powder spraying, wherein the powder according to the invention is sprayed with a powder jet device onto the tooth surfaces together with a gaseous carrier medium, in particular air.

The invention further relates to a process of cleaning tooth surfaces by applying the powder according to the invention to a tooth surface with a powder jet device.

The following examples provide preferred embodiments according to the invention and further exemplify the invention.

### Examples

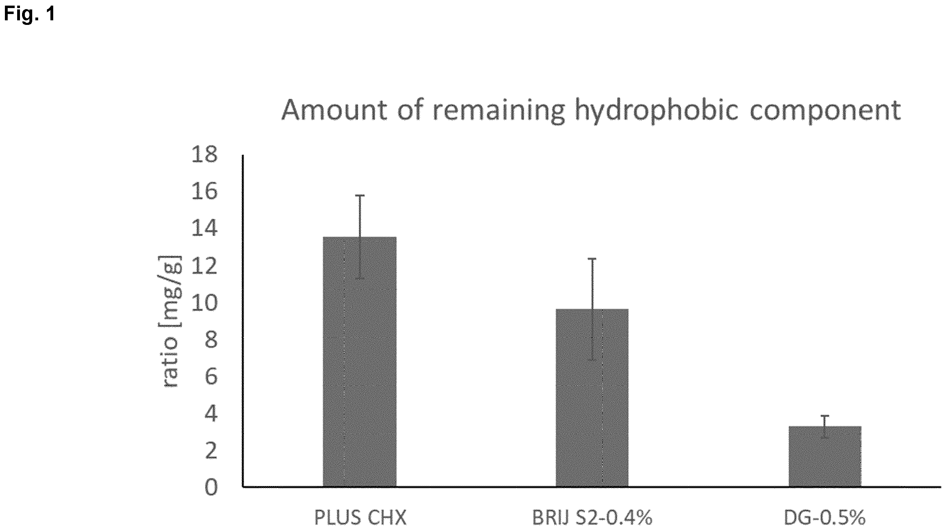

Fig. 1 shows the amount of non-dissolved (remaining) hydrophobic component according to the examples.
- Powder is sprayed tangentially in a dropping funnel during 1 minute with maximum water and standard setting (setting 3) of an AFPM (EMS) device
- After waiting 2 minutes, water and dissolved part is removed carefully from the bottom of the funnel
- The remaining residues are then dissolved in a 20% Isopropyl alcohol solution (15 mL)
- This solution is then filtrated on a 0.2 um CA filter paper which was initially weighted. The funnel is carefully washed with some water and then isopropyl alcohol to ensure no residues sticking on the walls.
- The filter paper is then dried (> 1 h @ 40°C) and the non-dissolved residue weighted

As powders EMS Plus CHX (a marketed product comprising erythritol as main component and chlorhexidine) is used without further additives and with 0.5 wt-% added DG (Decyl Glucoside) and BRIJ S2 0.4 wt-%.

Typical results graphs are shown in Fig. 1. The addition of the surfactant is reducing the amount of non-dissolved hydrophobic component.

## Claims

1. Powder for use in treating tooth surfaces with a powder jet device, wherein the powder comprises 0.1 - 2.0 wt.-% silica, based on the total weight of the powder, **characterized in that** the powder comprises 0.2 - 1.5 wt.-% of one or more surfactant(s), based on the total weight of the powder.

2. Powder according to claim, **characterized in that** the surfactant has a hydrophilic-lipophilic balance (HLB) of 3 - 20, preferably 9 - 16, wherein the HLB is calculated according to the formula HLB = 20 · Mₕ/M, wherein Mₕ is the molecular mass of the hydrophilic portion of the surfactant molecule, and M is the total molecular mass of the surfactant molecule.

3. Powder according to claim 1 or 2, **characterized in that** the surfactant is non-ionic.

4. Powder according to any of the preceding claims, **characterized in that** the surfactant contains a polyethylene glycol (PEG) and/or a glucoside.

5. Powder according to claim 4, **characterized in that** the surfactant is polyethylene glycol n-dodecylether according to the formula HO(CH₂CH₂O)ₚ-n-C₁₂H₂₅, wherein p = 2 - 10, and/or the surfactant is decyl glucoside.

6. Powder according to any of the preceding claims, **characterized in that** the powder comprises 0.2 - 1.8 wt.-% silica.

7. Powder according to any of the preceding claims, **characterized in that** the powder comprises 0.4 - 1.0 wt.-% of the one or more surfactant(s).

8. Powder according to any of the preceding claims, **characterized in that** the silica is a mixture of hydrophobic silica and hydrophilic silica in a mass ratio of 10:1 to 1:3.

9. Powder according to any of the preceding claims, **characterized in that** the powder has an average particle size (d₅₀) of 1 - 100 µm.

10. Powder according to any of the preceding claims, **characterized in that** the powder comprises 80 - 99 wt.-% of one or more components selected from the group consisting of sodium hydrogen carbonate, amino acids, alditols, sugars and cyclodextrins.

11. Powder according to any of the preceding claims, **characterized in that** the powder comprises 80 - 99 wt.-% of one or more components selected from the group consisting of sodium hydrogen carbonate, glycine, mannitol, erythritol, trehalose, tagatose, rhamnose and arabinose.

12. Powder according to any of the preceding claims, **characterized in that** the powder additionally comprises a bleaching agent, a bactericide, an analgesic and/or a flavouring agent.

13. Powder according to any of the preceding claims, **characterized in that** the powder comprises less than 0.3 wt.-%, preferably less than 0.1 wt.-% chlorhexidine, chlorhexidine digluconate and/or cetylpyridinium chloride, based on the total weight of the powder.

14. Powder according to any of the preceding claims, **characterized in that** the powder comprises less than 0.3 wt.-%, preferably less than 0.1 wt.-% bactericide, based on the total weight of the powder.

15. Use of a powder according to any of the preceding claims in a powder jet device.
